# EUROPEAN PATENT APPLICATION

(11) **EP 2 765 206 A1**
(43) Date of publication of application: **13.08.2014**
(21) Application number: 14162068.2
(22) Date of filing: 22.10.2008
(51) Int. Cl.: C12Q 1/68

(54) **Methods of performing ultra-sensitive immunoassays**

(30) Priority: 25.10.2007 US 923828
(62) Divisional of application: 08843235.6
(71) Applicant: Abbott Laboratories, Abbott Park, Illinois 60064-6008 (US)
(72) Inventor: Lou, Sheng, C., Lake Bluff, IL 60044-1147 (US); Chau, Kurt, H., Libertyville, IL 60048 (US); Konrath, John, G., Lake Villa, IL 60046 (US)
(74) Representative: Modiano, Micaela Nadia

(57) **Abstract**

A method for improving the sensitivity of an immunoassay by a factor of at least 10 to about 25, and perhaps greater. Furthermore, in addition to increasing the sensitivity of the assay, the method of this invention also improves assay specificity. In order to match or improve upon sensitivity of 0.2 pg/mL for an analyte, this invention provides an immunoassay involving amplification of a signal, e.g., a chemiluminescent signal, a specific binding member, e.g., a monoclonal antibody, and microparticle separation, e.g., magnetic microparticle separation, from large volumes of sample (e.g., from about 0.2 to about 3 mL). Such an immunoassay can be carried out with an automated immunoassay analyzers, e.g., an automated immunoassay analyzer in the ARCHITECT TM family of analyzers.

## Description

This application claims priority to U.S. Patent Application Serial No. 11/923,828, filed October 25, 2007, the contents of which are incorporated herein by reference.

### BACKGROUND OF THE INVENTION

### 1. Field of the Invention

This invention relates to immunoassays, and, more particularly to immunoassays for analytes that are present in low concentrations in samples.

### 2. Discussion of the Art

Nucleic acid testing can process samples having a volume of up to 1 milliliter (mL). Nucleic acid testing requires sample preparation steps to isolate RNA or DNA and 20 to 40 polymerase chain reaction (PCR) cycles to amplify the target. These steps require four to five hours to perform the assay. Nucleic acid testing provides higher sensitivity for detection than do automated immunoassay analyzers. However, nucleic acid testing is labor intensive, requires a long period of time to complete, and is expensive.

Most currently used automated immunoassay analyzers process samples having a volume of only about 100 microliters (µL), require less than one hour to complete, and are inexpensive. The sensitivity of detection in automated immunoassay analyzers is limited because (1) the small volume of sample used, e.g., from about 75 µL to about 200 µL, may contain smaller quantities of an analyte that can be detected by the analyzers, and (2) current analyzers cannot process larger samples, e.g., greater than 200 µL of sample.

It would be desirable to close the sensitivity gap between nucleic acid testing and testing by means of automated immunoassay analyzers, while retaining the speed of immunoassay testing and the lower cost associated with immunoassay testing.

The Food and Drug Administration, hereinafter alternately referred to as the FDA, has recently approved a nucleic acid tests for the screening of blood and plasma for such analytes as human immunodeficiency virus (HIV), hepatitis, and other viruses, all of which are present in biological fluids at low concentrations. Other analytes/antigens present in small concentrations in biological fluid include troponin, a cardiac biomarker for heart attack or heart damage, and thyroid stimulating hormone (TSH), an indicator for hyperthyroidism or hypothyroidism. Blood banks and plasma organizations are screening pools of donors for HIV and HCV by nucleic acid testing. Although implementation of nucleic acid testing is rapidly displacing HIV antigen testing, it would still be desirable to develop a low cost ultra-sensitive HIV (p24) antigen immunoassay for detection of antigen at 0.2 pg/mL or lower to compete with nucleic acid testing. An immunoassay sensitivity of 0.2 pg/mL is more sensitive than the nucleic acid sensitivity of 5,000 HIV copies of RNA/mL required by the Food and Drug Administration for nucleic acid testing systems.

### SUMMARY OF THE INVENTION

This invention provides a method for improving the sensitivity of an immunoassay by a factor of at least 10 to about 25, and perhaps greater. Furthermore, in addition to increasing the sensitivity of the assay, the method of this invention also improves assay specificity.

In order to match or improve upon sensitivity of 0.2 pg/mL for an analyte, this invention provides an immunoassay involving amplification of a signal, e.g., a chemiluminescent signal, a specific binding member, e.g., a monoclonal antibody, and microparticle separation, e.g., magnetic microparticle separation, from large volumes of sample (e.g., from about 0.2 mL to about 3 mL). Such an immunoassay can be carried out by using a KingFisher™ mL magnetic particle processor or a KingFisher™ magnetic particle processor as a sample processor to capture analytes from a large volume of sample combined with an automated immunoassay analyzer, e.g., an automated immunoassay analyzer in the ARCHITECT^{®} family of analyzers, commercially available from Abbott Laboratories, Abbott Park, Illinois. The method comprises the steps of:
(a) providing a plurality of containers;
(b) introducing a biological sample suspected of containing an analyte comprising a first specific binding member and magnetic microparticles coated with a second specific binding member into at least one container of the plurality of containers;
(c) allowing the first specific binding member to react with the second specific binding member to form a complex comprising the magnetic microparticles;
(d) collecting the complex formed in step (c) by means of inverse magnetic particle processing;
(e) releasing the complex collected in step (d) into a container of the plurality of containers containing wash buffer by means of inverse magnetic particle processing;
(f) washing the complex released in step (e) to wash away unbound first binding member and non-specifically bound components by means of inverse magnetic particle processing;
(g) collecting the complex washed in step (f) by means of inverse magnetic particle processing;
(h) introducing the complex into an automated immunoassay analyzer; and
(i) determining the concentration of the analyte in the sample by means of the automated immunoassay analyzer.

In a second embodiment, an automated analyzer is not required. In this second embodiment, an immunoassay can be carried out by a KingFisher™ mL magnetic particle processor or a KingFisher™ magnetic particle processor using a specific binding member to capture analyte from a large volume of sample, to then form a sandwich complex by means of a conjugate having a specific binding member, and then reading a signal generated by a moiety of the conjugate. The method comprises the steps of
(a) providing a plurality of containers;
(b) introducing a biological sample suspected of containing an analyte comprising a first specific binding member and magnetic microparticles coated with a second specific binding member into at least one container of the plurality of containers;
(c) allowing the first specific binding member to react with the second specific binding member to form a complex comprising the magnetic microparticles;
(d) collecting the complex formed in step (c) by means of inverse magnetic particle processing;
(e) releasing the complex collected in step (d) into a container of the plurality of containers, said container containing either (i) wash buffer but no third specific binding member or (ii) a third specific binding member, said releasing carried out by means of inverse magnetic particle processing;
(f) if the container in step (e) contains the wash buffer but does not contain the third specific binding member, adding the third specific binding member to the container of step (e);
(g) allowing the third specific binding member to react with the complex released in step (e) to form a sandwich complex;
(h) collecting the sandwich complex formed in step (g) by means of inverse magnetic particle processing;
(i) releasing the sandwich complex collected in step (h) into a container of the plurality of containers, which container contains either (i) wash buffer, to wash away unbound third specific binding member, or (ii) sample, to wash away unbound third specific binding member, said releasing carried out by means of inverse magnetic particle processing;
(j) collecting the sandwich complex released in step (i) by means of inverse magnetic particle processing;
(k) releasing the sandwich complex collected in step (j) into a container of the plurality of containers by means of inverse magnetic particle processing, which container contains pre-trigger solution, thereby releasing the specifically bound third binding member;
(l) collecting the magnetic particles from the pre-trigger solution by means of inverse magnetic particle processing and releasing the magnetic microparticles into a container of the plurality of containers, which container contains wash buffer; and
(m) determining the concentration of the analyte in the sample by injecting trigger solution into the pre-trigger solution and reading the signal generated.

The method described herein increases the sensitivity of an immunoassay by increasing the amount of analyte available for use in the immunoassay. The method described herein improves the sensitivity of an assay to a level that is close to, i.e., substantially equivalent to the sensitivity of nucleic acid testing, i.e., molecular diagnostics.

### BRIEF DESCRIPTION OF THE DRAWINGS

FIG. 1 is a perspective view of a KingFisher™ mL magnetic particle processor.
FIG. 2 is a front view in elevation illustrating a KingFisher™ mL magnetic particle processor suitable for carrying out the procedure of inverse magnetic particle processing to prepare a sample for an immunoassay.
FIG. 3 is a front view in elevation illustrating a KingFisher™ magnetic particle processor suitable for carrying out the procedure of inverse magnetic particle processing to prepare a sample for an immunoassay. This processor utilizes micro-well plates having 96 micro-wells per micro-well plate.
FIG. 4 is a top view of a micro-well plate suitable for carrying out the procedure of inverse magnetic particle processing to prepare a sample for an immunoassay. In FIG. 4, two well strips are removed from the micro-well plate. One of the removed well strips can be seen in a top view format. The other of the removed well strips can be seen in a side elevational view format.
FIG. 5 is a side view in elevation of a tip comb suitable for use with a KingFisher™ magnetic particle processor.
FIGS. 6A, 6B, 6C, 6D, 6E, and 6F constituting a series of schematic diagrams illustrating the procedure of inverse magnetic particle processing utilized by a KingFisher™ mL magnetic particle processor.
FIGS. 7A, 7B, 7C, 7D, 7E, and 7F constituting a series of schematic diagrams illustrating the procedure of inverse magnetic particle processing utilized by a KingFisher™ magnetic particle processor.
FIG. 8 is a graph illustrating signal enhancement resulting from utilizing the procedure of inverse magnetic particle processing to prepare a sample for an immunoassay. FIG. 8 shows a plot of signal-to-noise ratio for samples analyzed in an ARCHITECT^{®} automated immunoassay analyzer both without previous treatment by inverse magnetic particle processing (indicated by squares) and with previous treatment by inverse magnetic particle processing (indicated by diamonds).

### DETAILED DESCRIPTION

As used herein, the term "container" is intended to include both tubes and wells. The term "well" includes micro-wells and wells having greater volume than a micro-well.

As used herein, the expressions "label", "label group", and the like mean a group attached to a specific binding member, e.g., an antibody or an antigen, to render the reaction between the specific binding member and its complementary binding member detectable. Representative examples of labels include enzymes, radioactive labels, fluorescein, and chemicals that produce light. A label is any substance that can be attached to an immunoreactant and that is capable of producing a signal that is detectable by visual or instrumental means. Various labels suitable for use in this invention include catalysts, enzymes, liposomes, and other vesicles containing signal producing substances such as chromogens, catalysts, fluorescent compounds, chemiluminescent compounds, enzymes, and the like. A number of enzymes suitable for use as labels are disclosed in U. S. Patent No. 4,275,149, incorporated herein by reference. Such enzymes include glucosidases, galactosidases, phosphatases and peroxidases, such as alkaline phosphatase and horseradish peroxidase, which are used in conjunction with enzyme substrates, such as fluorescein di(galactopyranoside), nitro blue tetrazolium, 3,5',5,5'-tetranitrobenzidine, 4-methoxy-1-naphthol, 4-chloro-1-naphthol, 4-methylumbelliferyl phosphate, 5-bromo-4- chloro-3-indolyl phosphate, chemiluminescent enzyme substrates, such as the dioxetanes described in WO 88100694 and EP 0-254-051-A2, and derivatives and analogues thereof. Preferably, the label is an enzyme and most preferably the enzyme is alkaline phosphatase.

As used herein, the expression "test sample", the expression "biological sample", and the term "sample" refer to a material suspected of containing an analyte. The test sample can be used directly as obtained from the source or following a pretreatment to modify the character of the sample. The test sample can be derived from any biological source, such as a physiological fluid, such as, for example, blood, saliva, ocular lens fluid, cerebral spinal fluid, sweat, urine, milk, ascites fluid, synovial fluid, peritoneal fluid, amniotic fluid, and the like. The test sample can be pretreated prior to use, such as preparing plasma from blood, diluting viscous fluids, and the like. Methods of treatment can involve filtration, distillation, extraction, concentration, inactivation of interfering components, the addition of reagents, and the like. Other liquid samples besides physiological fluids can be used, such as water, food products, and the like, for the performance of environmental or food production assays. In addition, a solid material suspected of containing the analyte can be used as the test simple. In some instances it may be beneficial to modify a solid test sample to form a liquid medium or to release the analyte.

As used herein, the expression "specific binding member" means a member of a specific binding pair, i.e., two different molecules where one of the molecules through chemical or physical means specifically binds to the second molecule. An example of such specific binding members of a specific binding pair is an antigen and an antibody that specifically binds to that antigen. Another example of such binding members of a specific binding pair is a first antibody and a second antibody that specifically binds to the first antibody.

As used herein, the term "conjugate" means a binding member, e.g., an antigen or an antibody, coupled to a detectable moiety.

As used herein, the expressions "solid phase", "solid phase material", and the like, mean any material that is insoluble, or can be made insoluble by a subsequent reaction. Representative examples of solid phase material include polymeric or glass beads, microparticles, tubes, sheets, plates, slides, wells, tapes, test tubes, or the like.

As used herein, the term "analyte" means the compound to be detected or measured. The analyte has *at* least one epitope or binding site.

As used herein, the expression "monoclonal antibodies" means antibodies that are identical because they were produced by one type of immune cell and are all clones of a single parent cell.

As used herein, the term "binding affinity of an antibody" means the strength of the interaction between a single antigen-binding site on an antibody and its specific antigen epitope. The higher the affinity, the tighter the association between antigen and antibody, and the more likely the antigen is to remain in the binding site. The affinity constant is the ratio between the rate constants for binding and dissociation of antibody and antigen. Typical affinities for IgG antibodies are 10⁵ to 10⁹ L/mole.

As used herein, the expression "normal human plasma" means human plasma that is free of the analyte of interest or other known abnormality or pathology.

As used herein, the symbol "(s)" following a noun means one or more of the noun, depending upon the context.

As used herein, the term "pre-activated" means reacting 1-ethyl-3-(3-dimethylaminopropyl)carbodi-imide hydrochloride (hereinafter "EDAC") and N-hydroxysulfosuccinimide (hereinafter "sulfo-NHS") with the carboxyl groups on microparticles to provide semi-stable NHS esters that will react with NH₂ groups on monoclonal antibodies to form stable amide bonds that couple the antibodies to the microparticles.

As used herein, the term "magnetic microparticles" means paramagnetic microparticles. Paramagnetic microparticles are attracted to magnetic fields, hence have a relative magnetic permeability greater than one. However, unlike ferromagnets, which are also attracted to magnetic fields, paramagnetic materials do not retain any magnetization in the absence of an externally applied magnetic field.

As used herein, the symbol "(s)" following the name of an item indicates that one or more of the subject items is intended, depending upon the context. As used herein, the symbol "S/N" means signal to noise ratio.

As used herein, the term "immunoassay" means a special class of assay or test that is performed in a container, e.g., a test tube, which assay or test uses an antibody-antigen reaction to determine whether a patient has been exposed to the antigen or has an antibody to the antigen. An immunoassay can be a heterogeneous immunoassay or a homogeneous immunoassay. The method described herein is primarily concerned with the heterogeneous immunoassay.

Heterogeneous immunoassays can be performed in a competitive immunoassay format or in a sandwich immunoassay format. In the competitive immunoassay format, an antigen can be immobilized to a solid phase material. The amount of detectable moiety that binds to the solid phase material can be detected, measured, and correlated to the amount of antibody (antigen) present in the test sample. Examples of solid phase materials include beads, particles, microparticles, and the like.

The present invention is concerned primarily with the sandwich immunoassay format. In the sandwich assay immunoassay format, a solid phase, e.g., a microparticle, is coated with antibodies. The antibody on the solid phase is known as the capture antibody. The assay is intended to detect and measure antigens in the sample. A second antibody is labeled with an appropriate label, e.g., acridinium. The second antibody is not attached to a solid phase. The second antibody is known as the detection antibody. The antibody and antigen attach in the following order: antibody on solid phase-antigen-antibody having a label. The solid phase is removed. The antibody-antigen-antibody sandwich enables measurement of the antigen by activating the label, which can be used to determine the concentration of analyte in the sample. As used herein, the expression "sandwich complex" means an antibody-antigen-antibody sandwich.

In one example of the sandwich immunoassay format, a test sample containing an antibody is contacted with an antigen, e.g., a protein that has been immobilized on a solid phase material thereby forming an antigen-antibody complex. Examples of solid phase materials include beads, particles, microparticles, and the like. The solid phase material containing the antigen-antibody complex is typically treated for example with a second antibody that has been labeled with a detectable moiety. The second antibody then becomes bound to the antibody of the sample that is bound to the antigen immobilized on the solid phase material. Then, after one or more washing steps to remove any unbound material, an indicator material, such as a chromogenic substance, is introduced to react with the detectable moiety to produce a detectable signal, e.g. a color change. The color change is then detected, measured, and correlated to the amount of antibody present in the test sample. It should also be noted that various diluents and buffers are also required to optimize the operation of the microparticles, antigens, conjugates and other components of the assay that participate in chemical reactions. It should be further noted that other types of sandwich assays can be utilized, such as, for example, where the first antibody is immobilized on the solid phase material.

A heterogeneous immunoassay to determine the concentration of an analyte present at a low concentration in a biological sample can be performed with the apparatus described in United States Patent Nos. 5,795,784 and 5,856,194, in a sandwich immunoassay format, which employs microparticles as the solid phase material. These patents are incorporated herein by reference.

In the case of HIV antigens, such as, for example, HIV-1 p24 antigen, it is preferred that monoclonal antibodies be used to carry out the immunoassay described herein. For example, monoclonal antibodies 120A-270 and 1158-151 can be used as a component of a solid phase capture antibody and as a detection antibody conjugate, respectively, to develop an ultra-sensitive immunoassay for HIV-1 p24 antigen for use in commercially available automated immunoassay analyzers. These monoclonal antibodies are described in greater detail in U. S. Patent No. 6,818,392, incorporated herein by reference. Monoclonal antibodies are typically selected on the basis of their high binding affinities (e.g., greater than 5 x 10⁹ liters/mole), compatibility between components for sandwich assays, and detection of all subtypes of the antigen tested. The monoclonal antibodies for the HIV-1 p24 antigen mentioned previously can be used to determine all subtypes of HIV-1 p24 antigen and HIV-2 p26 antigen.

Prior to carrying out an immunoassay for antigens that are present at a concentration of lower than about 0.2 pg/mL, the method described herein utilizes a processing technique to prepare biological samples for use in a commercially available automated immunoassay analyzer. Such a processing technique can be carried out with a KingFisher™ mL magnetic particle processor or a KingFisher™ magnetic particle processor, both of which are commercially available from Thermo Fisher Scientific, Inc., Waltham, MA.

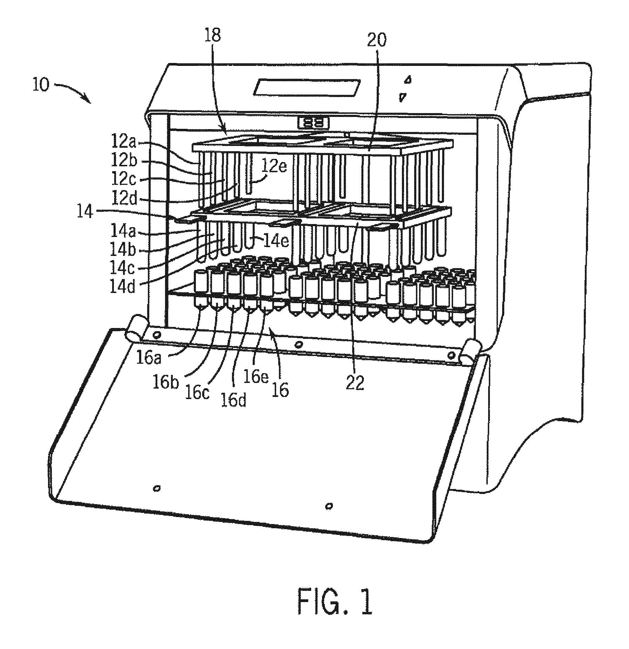

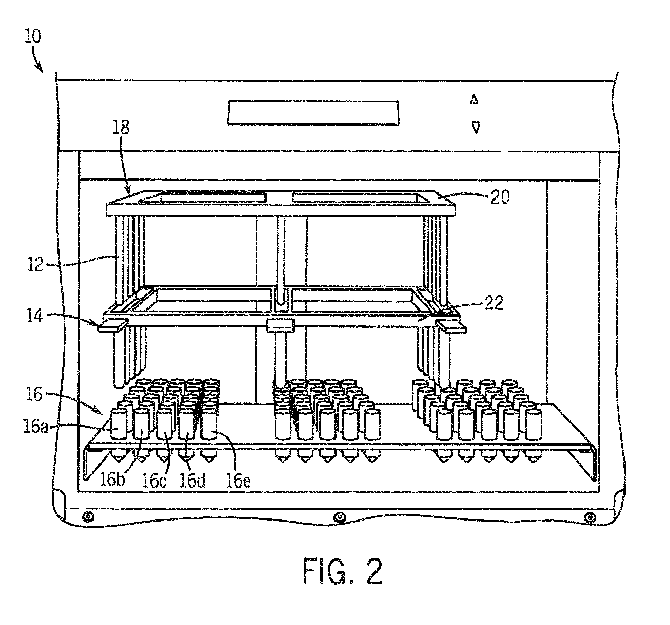

Referring now to FIGS. 1 and 2, a KingFisher™ mL magnetic particle processor 10 can be used for automated transfer and processing of magnetic particles in tubes of a tube strip. In the description that follows, the tubes of the tube embodiment will be used to illustrate the concentrating technique. The principle of the KingFisher™ mL magnetic particle processor 10 is based on the use of (a) magnetic rods 12a, 12b, 12c, 12d, and 12e that can be covered with disposable tip combs 14 and (b) tube strips 16. A tip comb 14 comprises a strip of non-magnetic material that joins a plurality of sheaths 14a, 14b, 14c, 14d, and 14e made of non-magnetic material for covering magnetic rods. A tube strip 16 is a plurality of tubes 16a, 16b, 16c, 16d, and 16e arranged in a row. The KingFisher™ mL magnetic particle processor 10 is capable of functioning without any aspiration and/or dispensing devices. The KingFisher™ mL magnetic particle processor 10 is designed for a maximum of fifteen (15) tube strips 16, which are compatible with the tip comb 14. The tube strip(s) 16 is (are) maintained stationary and the only movable assembly is a processing head 18 along with the tip combs 14 and magnetic rods 12a, 12b, 12c, 12d, and 12e associated therewith. The processing head 18 comprises two vertically moving platforms 20, 22. One platform 20 is needed for the magnetic rods 12a, 12b, 12c, 12d, and 12e, and the other platform 22 is needed for the tip combs 14. A tray 24 contains 15 separate tube strips 16 and a single sample processing typically uses one tube strip 16 containing five tubes 16a, 16b, 16c, 16d, and 16e. One tip comb 14 containing five tips 14a, 14b, 14c, 14d, and 14e is used for processing five samples at one time.

Before starting the magnetic particle processing via a keypad (not shown) and a display (not shown), the samples and reagents are dispensed into the tubes 16a, 16b, 16c, 16d, and 16e and the tip comb(s) 14 is (are) loaded into its (their) slot(s). The tube strip(s) 16 is (are) placed into the removable tray in the correct position and the tray is pushed into the end position. During the operation, the front and top lids can be closed or open. Closed lids protect the processing against environmental contamination. The KingFisher™ mL magnetic particle processor is described in detail in KingFisher™ mL User manual, Revision No. 1.0, February 2002, Catalog No. 1508260, incorporated herein by reference.

The KingFisher™ magnetic particles processor is designed for the automated transfer and processing of magnetic particles in volumes of liquids suitable for micro-wells. This is in contrast to the KingFisher™ mL magnetic particle processor, which employs greater volumes of liquids. The KingFisher™ magnetic particle processor is described in detail in KingFisher™ Micro-well User Manual, Revision No. 1.0, 1999-04-09, Catalog No. 1507730, incorporated herein by reference.

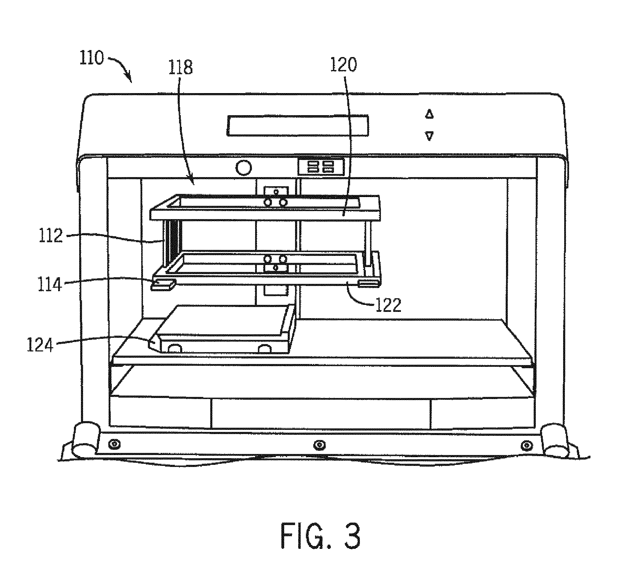

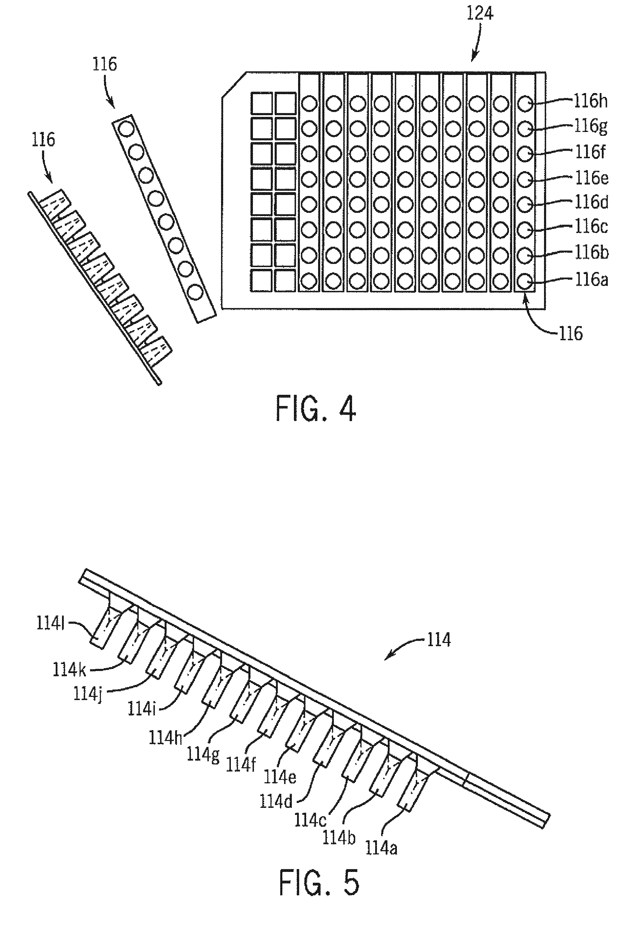

Referring now to FIGS. 3, 4, and 5, a KingFisher™ magnetic particle processor 110 can be used for automated transfer and processing of magnetic particles in wells of a micro-well plate. The principle of the KingFisher™ magnetic particle processor 110 is based on the use of magnetic rods 112a, 112 b, 112c, 112d, 112e, 112f, 112g, 112h that can be covered with disposable tip combs 114 and well strips 116. Only the magnetic rod 112a is shown. The other magnetic rods are hidden by the magnetic rod 112a. A tip comb 114 comprises a strip of non-magnetic material that joins a plurality of sheaths made of non-magnetic material for covering magnetic rods. A well strip 116 is a plurality of micro-wells arranged in a row. The KingFisher™ magnetic particle processor 110 is capable of functioning without any aspiration and/or dispensing devices. The KingFisher™ magnetic particle processor 110 is designed for a maximum of ninety-six (96) micro-wells, which are compatible with the tip comb 114. The micro-wells are maintained stationary and the only movable assembly is a processing head 118 along with the tip combs 114 and magnetic rods 112 associated therewith. The processing head 118 comprises two vertically moving platforms 120, 122. One platform 120 is needed for the magnetic rods 112 and the other platform 122 is needed for the tip combs 114. A tray 124 contains one micro-well plate and a single sample processing typically uses one well strip 116 containing eight micro-wells 116a, 116b, 116c, 116d, 116e, 116f, 116g, and 116h. One tip comb 114 containing twelve tips 114a, 114b, 114c, 114d, 114e, 114f, 114g, 114h, 114i, 114j, 114k, and 114l is used for processing twelve samples at one time.

Before starting the magnetic particle processing via a keypad (not shown) and a display (not shown), the samples and reagents are dispensed into the micro-wells 116a, 116b, 116c, 116d, 116e, 116f, 116g, and 116h and the tip comb(s) 114 is (are) loaded into its (their) slot(s). The well strip(s) 116 is (are) placed into the removable tray in the correct position and the tray is pushed into the end position. During the operation, the front and top lids can be closed or open. Closed lids protect the processing against environmental contamination.

Regardless of which of the aforementioned KingFisher™ instrument is being used, the operating principle employed is inverse magnetic particle processing technology, commonly referred to as MPP. Rather than moving the liquids from one well to another, the magnetic particles are moved from the tube 16a (or from the micro-well 116a) to the tube 16b (or to the micro-well 116b), at least one tubes (micro-wells) containing specific reagent(s). This principle stands in contrast to the external magnet method, i.e., the type of separation used in the apparatus shown in U. S. Patent Nos. 5,795,784 and 5,856,194. According to inverse magnetic particle processing technology, magnetic particles are transferred with the aid of magnetic rods covered with disposable, specially designed plastic tip combs. Working with magnetic particles can be divided into five separate process steps:
Collecting particles: in this step, magnetic particles are collected from the well or tube specified.
Binding particles: In this step, material is collected onto the magnetic particles from the reagent in a specific well or tube.
Mixing particles: In this step, the reagent and particles (if inserted), are mixed with the plastic tip in a specific well or tube.
Releasing particles: In this step, the collected material is released from the surfaces of the magnetic particles into a specific well or tube.
Washing particles: In this step, the magnetic particles are washed in a specific well or tube.

During the collection of the magnetic particles, the magnetic rod is fully inside the tip. The magnetic rods together with the tip comb(s) move slowly up and down in the tubes and the magnetic particles are collected onto the wall of the tips. The magnetic rods together with the tip comb(s), having collected the magnetic particles, can be lifted out of the tubes and transferred into the next tubes. After collection of the magnetic particles, the magnetic rods together with the tip comb(s) are lifted from the tubes, the magnetic rods are lifted off and the tip comb(s) is (are) lowered into the next tubes containing a reagent. Magnetic particles are released by moving the tip comb(s) up and down several times at considerably high speed until all the particles have been mixed with the substance in the next reaction. Washing the magnetic particles is a frequent and an important processing phase. Washing is a combination of the release and collection processes in a tube filled with washing solution. To maximize washing efficiency, the magnetic rods together with the tip comb(s) are designed to have minimized liquid-carrying properties. To keep the magnetic particle suspension evenly mixed in long-running reactions, the tip comb(s) can be moved up and down from time to time. The volume of the first tube can be larger than the volume of the next tube and this is used for concentration purposes.

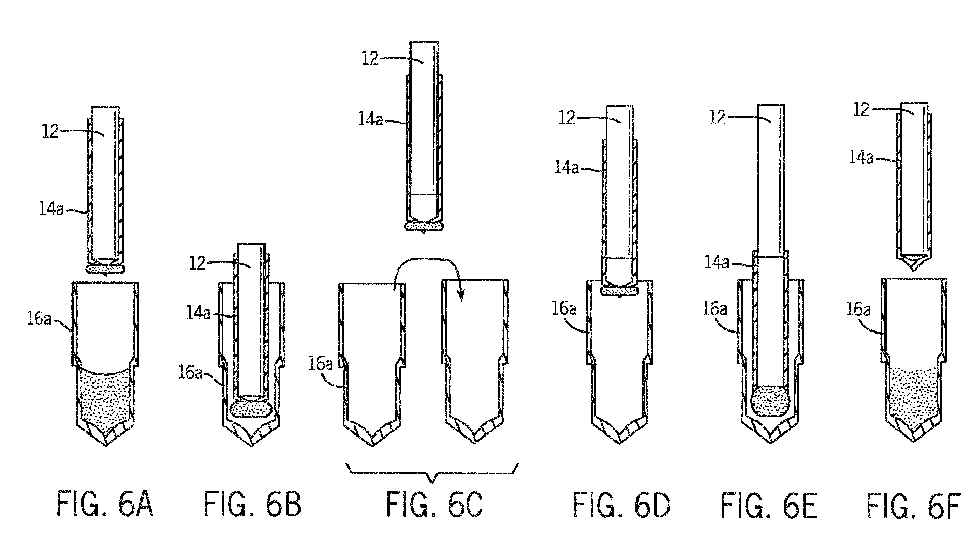

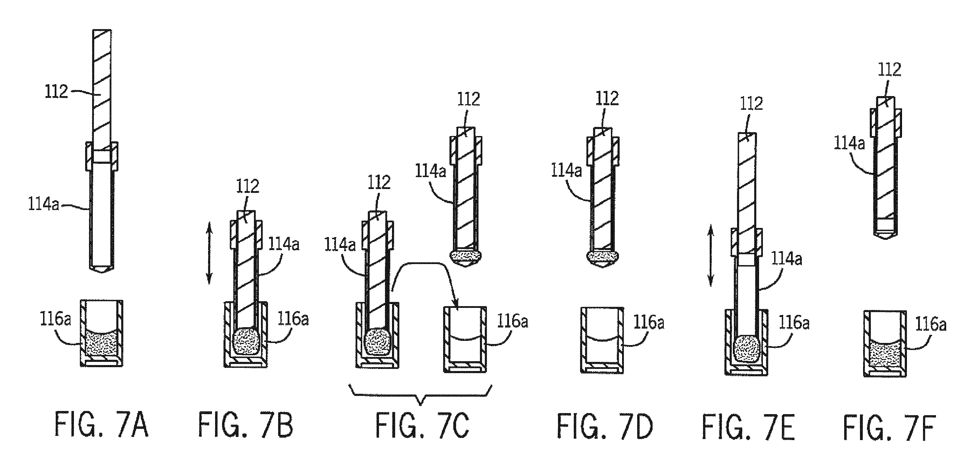

FIGS. 6A, 6B, 6C, 6D, 6E, and 6F illustrate the sequence of steps employed in collecting, transferring, and releasing magnetic particles from tubes in a KingFisher™ mL magnetic particle processor. FIGS. 7A, 7B, 7C, 7D, 7E, and 7F illustrate the sequence of steps employed in collecting, transferring, and releasing magnetic particles from micro-wells in a KingFisher™ magnetic particle processor.

The method described herein makes it possible to detect analytes by means of an immunoassay at a level equivalent to the FDA standard of at least 5,000 RNA copies/mL for testing individual sera or plasma. See Guidance for Industry In the Manufacture and Clinical evaluation if In Vitro Tests to Detect Nucleic Acid Sequences of Human Immunodeficiency Viruses Types 1 and 2, U. S. Department of Health and human Services, Food and Drug Administration, Center for Biologics Evaluation and Research (CBER), December 1999. In addition, the method described herein makes it possible to detect analytes by means of an immunoassay at a level equivalent to the FDA standard of at least 100 RNA copies/mL for testing pooled sera or plasma. See Guidance for Industry In the Manufacture and Clinical evaluation if In Vitro Tests to Detect Nucleic Acid Sequences of Human Immunodeficiency Viruses Types 1 and 2, U. S. Department of Health and human Services, Food and Drug Administration, Center for Biologics Evaluation and Research (CBER), December 1999. Such features match or exceed the sensitivity standards of the FDA when the HIV-1 p24 antigen is used as the detection target instead of RNA. TABLE I lists the characteristics of nucleic acid testing for HIV with testing for HIV-1 p24 antigen by means of a PRISM^{®} analyzer, commercially available from Abbott Laboratories, Abbott Park, Illinois, and with testing for HIV-1 p24 antigen by means of the method described herein.

**TABLE I**

| Feature | Nucleic acid testing for HIV | Testing for HIV-1 p24 antigen by means of "PRISM" automated analyzer | Testing for HIV-1 p24 antigen by the method described herein |
|---|---|---|---|
| Detected target | RNA | p24 protein | p24 protein |
| Assay sensitivity | 100 RNA copies/mL | 5 pg/mL (10,000 RNA copies) | 0.2 pg/mL (400 RNA copies) |
| Volume of sample | 1 mL | 0.1 mL | 1 mL |
| Total time of assay | 5 hours | 1 hour | 2 hours |
| Time of sample preparation | 1 hour | N/A | 1 hour |
| Amplification | 36 PCR cycles (3 hours) | N/A | Signal amplification |
| Time of detection | 1 hour | 1 hour | 1 hour |

### EXAMPLES

In these examples, the term "SPSP-Acr-115B-151-423" means a conjugate comprising the anti-HIV-1 p24 monoclonal antibody 115B-151-423 and *N*¹⁰-(3-sulfopropyl)-*N*-(3-sulfopropyl)-acridinium-9-carboxamide. The monoclonal antibody 115B-151-423 is a monoclonal antibody specific for the HIV-1 p24 antigen. The term "CPSP-Acr-115B-151-423" means a conjugate comprising the anti-HIV-1 p24 monoclonal antibody 115B-151-423 and *N*¹⁰-(3-sulfopropyl)-*N*-(3-carboxypropyl)-acridinium-9-carboxamlde. The term "anti-HIV-1 p24 monoclonal antibody 120A-270-1068" means a monoclonal specific for the HIV-1 p24 antigen. The term "anti-HIV-1 p24 monoclonal antibody 108-394-470" means a monoclonal antibody specific for the HIV-1 p24 antigen.

In these examples, products having the trademarks ARCHITECT^{®} and PRISM^{®} are commercially available from Abbott Laboratories, Abbott Park, Illinois.

The following preparatory examples illustrate the preparation of magnetic particles containing the anti-HIV-1 p24 monoclonal antibody 120A-270-1068 and the preparation of a conjugate comprising the anti-HIV-1 p24 monoclonal antibody 115B-151-423 and an acridinium label.

### PREPARATION A

The following materials were used to prepare magnetic particles containing the anti-HIV-1 p24 monoclonal antibody 120A-270-1068:
1. Magnetic microparticles (Magnetic CML azo-valeric, 4.7 micrometers, 5.1% solids, Polymer Science)
2. Anti-HIV-1 p24 monoclonal antibody 120A-270-1068 (3.4 mg/mL)
3. Coating buffer (MES buffer, 50 mM, pH 6.1, 0.2 micrometer filtered)
4. EDAC (2.5 mg/mL in 50 mM MES buffer, pH 6.1)
5. Sulfo-NHS (2.5 mg/mL in 50 mM MES buffer, pH 6.1, molecular weight 217.14, Pierce Chemical)
6. Phosphate buffered saline (10 mM phosphate buffer, pH 7.2 with 0.15 M sodium chloride)
7. Overcoat buffer (0.1 M Tris buffer plus 1% bovine serum albumin, pH 7.2)
8. Polyethylene glycol (Molecular weight 8000, 10% w/v in distilled water)

The following method was used to coat magnetic microparticles (25 mL, 1% solids) with the anti-HIV-1 p24 monoclonal antibody 120A-270-1068 (100 µg/mL) in a two-step method. EDAC (at a concentration of 0.1 mg/mL) and sulfo-NHS (at a concentration of 0.1 mg/mL) were used to pre-activate the magnetic microparticles in a polypropylene tube (50 mL). The materials used in the method are listed above.
1. MES buffer (20.1 mL) was added to a polypropylene tube (50 mL).
2. Magnetic microparticles (4.9 mL) were added to the tube to yield a solution containing microparticles (25 mL, 1% solids).
3. The tube was rotated about its axis to wash the microparticles.
4. The microparticles were spun down at 3300 rpm for 10 minutes, and the supernatant fluid was aspirated.
5. The microparticles were washed two more times by adding MES buffer (25 mL) to resuspend the microparticles, the microparticles were spun at 3300 rpm for 10 minutes, and the supernatant fluid was aspirated.
6. The microparticles were resuspended in MES buffer (23 mL).
7. EDAC (1 mL) and sulfo-NHS (1 mL) were added to the tube to pre-activate the microparticles for 30 minutes at room temperature. Mixing was carried out on an end-over-end rotator (Roto-Torque (Cole Parmer) set at the high speed switch and at "6" on the speed dial, which ranged from 1 to 10).
8. The microparticles were spun down at 3300 rpm for 10 minutes, and the supernatant fluid was aspirated.
9. MES buffer (25 mL) was added to wash the microparticles one time, and the microparticles were spun down at 3300 rpm for 10 minutes, and the supernatant fluid was aspirated.
10. Anti-HIV-1 p24 monoclonal antibody 120A-270-1068 (25 mL at a concentration of 100 µg/mL) was added to the pellet containing the pre-activated microparticles.
11. The tube was placed on an end-over-end rotator (Roto-Torque (Cole Parmer) set at the high speed switch and at "6" on the speed dial, which ranged from 1 to 10) and rotated for 90 minutes at room temperature.
12. The microparticles were spun down at 3300 rpm for 10 minutes, and the supernatant fluid was aspirated.
13. Phosphate buffered saline (25 mL) was added to wash the microparticles to resuspend the microparticles. The microparticle were spun down at 3300 rpm for 10 minutes, and the supernatant fluid was aspirated. This wash step was carried out three times. For each wash step, 25 mL of phosphate buffered saline was used.
14. Overcoat buffer (22.5 mL) and polyethylene glycol (2.5 mL, 10% polyethylene glycol solution) were added to the microparticles.
15. The tube was rotated on an end-over-end rotator (Roto-Torque (Cole Parmer) set at the high speed switch and at "5" on the speed dial, which ranged from 1 to 10) for 16 hours at room temperature.
16. The microparticles were spun down at 3300 rpm for 10 minutes, and the supernatant fluid was aspirated.
17. The microparticles were resuspended by adding overcoat buffer (22.5 mL) and polyethylene glycol (2.5 mL, 10% solution of polyethylene glycol).
18. The microparticles coated with the anti-HIV-1 p24 monoclonal antibody 120A-270-1068 were stored at a temperature of 4 °C.

Substantially the same method as described above was used to prepare other monoclonal antibodies that were used in the working examples. For example, another antibody utilized in the working examples was the anti-HIV-1 p24 monoclonal antibody 108-394-470.

### PREPARATION B

The following materials were used to prepare the SPSP-Acr-115B-151-423 conjugate comprising the anti-HIV-1 p24 monoclonal antibody 1158-151-423 and an acridinium label:
1. Stock solution of the anti-HIV-1 p24 monoclonal antibody 115B-151-423 (9.7 mg/mL in 10 mM phosphate buffered saline, pH 7.25)
2. Pentfluorophenyl ester derivatized from *N*¹⁰-(3-sulfopropyl)-*N*-(3-sulfopropyl)-acridinium-9-carboxamide in dimethyl formamide (46.6 mM of the active ester in dimethyl formamide solvent)
3. HPLC grade water
4. Buffer solution, pH 4.0
5. Buffer solution, pH 7.0
6. Buffer solution, pH 10.0
7. NaCl (1.0M)
8. NaOH (1.0 N)
9. NaOH (6.0 N)
10. Na₂HPO₄·7H₂O
11. NaH₂PO₄ · H₂O
12. NaCl
13. 3-[(3-cholamidopropyl)-dimethylammonio]-1-propanesulfonate (CHAPS)
14. CHAPS (0.1 %CHAPS in phosphate buffered saline, pH 6.3)
15. Sodium phosphate buffer (0.1 M, 150 mM NaCl, pH 8.0)
16. Sodium phosphate buffer (1 M, 150 mM NaCl, pH 8.0)

The pentafluorophenyl ester derivatized from *N*¹⁰-(3-sulfopropyl)-*N*-(3-sulfopropyl)-acridinium-9-carboxamide is shown in Structure 1.

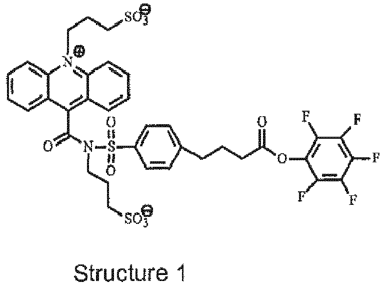

The following method was used to prepare a conjugate containing the anti-HIV-1 p24 monoclonal antibody 115B-151-423 and an acridinium label. The materials used in the method are listed above.
1. To the anti-HIV-1 p24 monoclonal antibody 115B-151-423 stock solution were added sodium phosphate buffer (0.10 mL, 1 M), and NaCl (150 mM, pH 8.0). The resultant mixture was filtered by means of a Millex-GV filter (0.22 µm). The final pH was adjusted to 8.0 with NaOH (about 20 µL, 0.1 N). The antibody (20 µL) was diluted with phosphate buffered saline (480 µL, 100 mM, pH 8.0 buffer). Absorbance at 280 nm was measured to determine the concentration of antibody. Phosphate buffered saline (100 mM, pH 8.0 buffer) was used as the blank. The concentration of the antibody stock solution was 25X diluted, i.e., 20 µL/500 µL) for the measurement. The concentration of anti-HIV-1 p24 monoclonal antibody 115B-151-423 stock solution was 9.0 mg/mL.
2. To each of four polypropylene microcentrifuge tubes (1.5 mL) were added the anti-HIV-1 p24 monoclonal antibody 115B-151-423 stock solution (0.222 mL, 2.0 mg), sodium phosphate (0.178 mL, 100 mM), NaCl (150 mM), conjugation buffer (pH 8.0) to make a solution (5 mg/mL). The solution was stirred with a spin bar (1 inch x 5/16 inch) at 800 revolutions per minute. Then the pentafluorophenyl ester derivatized from *N*¹⁰-(3-sulfopropyl)-*N*-(3-sulfopropyl)-acridin!um-9-carboxamide (40 mg/mL stock solution) was added as the solution was stirred. The reaction proceeded at ambient temperature in the dark for three (3) hours.

The following table indicates the quantities of materials that were combined to produce the conjugates.

**TABLE II**

| Conjugate | Antibody stock (µL) | Sodium phosphate (µL) | Pentafluorophenyl ester derivatized from *N*¹⁰-(3-sulfopropyl)-*N-*(3-sulfopropyl)-acridinium-9-carboxamide (µL) |
|---|---|---|---|
| A | 222.0 | 178.0 | 5.4 |
| B | 222.0 | 178.0 | 6.8 |
| C | 222.0 | 178.0 | 8.0 |
| D | 222.0 | 178.0 | 9.2 |

3. To HPLC grade water (4.0 L) were added sodium phosphate dibasic (10.3 g), sodium phosphate monobasic (0.268 g), sodium chloride (35.1 g), and CHAPS (4 g, pH 8.0) to prepare dialysis buffer. The pH was 8.0.
4. After the conjugation reaction, each conjugate was immediately injected into a Slide-A-Lyzer 10K Dialysis Cassette (0.5 - 3.0 mL) with a 1 cc Tuberculin Syringe (B-D Rec. No. 309602, Lot No. 2124269) equipped with Precision Glide 18G1 syringe needle. All four cassettes were placed into a beaker containing dialysis buffer (1.6 L). Dialysis proceeded at ambient temperature at a stirring rate of 350 revolutions per minute. Dialysis was carried out for 1.0 hour, then repeated for 2.0 hours each time with fresh phosphate buffered saline (10 mM, pH 8.0 buffer) and overnight, with fresh CHAPS (0.1 %)/ phosphate buffered saline (10 mM) pH 8.0.
5. HPLC purification buffer used in the HPLC purification procedure was CHAPS (0.1%)/10 mM phosphate buffered saline, pH 6.3. Each conjugate was injected into a TosoHaas G3000SW column (21.5 mm x 60 cm) with a single shot. The conjugate was eluted with HPLC purification buffer at a flow rate of 5.0 mL/min. for 60 minutes. The fractions were collected from 18 minutes to 30 minutes, each fraction being collected for 30 seconds. The fractions 10 through 20, inclusive, were combined.

The following table indicates the quantity of acridinium incorporated into the conjugates.

**TABLE III**

| Conjugate | A280 | A370 | Antibody (mg/mL) | Antibody (µM) | Acridinium (µM) | Acridinium/Antibody Ratio | Yield |
|---|---|---|---|---|---|---|---|
| A | 0.3991 | 0.2024 | 0.2596 | 1.7310 | 13.2217 | 7.6 | 0.52 |
| B | 0.4169 | 0.2641 | 0.2635 | 1.7569 | 17.3591 | 9.9 | 0.59 |
| C | 0.4379 | 0.3128 | 0.2716 | 1.8110 | 20.6175 | 11.4 | 0.61 |
| C | 0.3473 | 0.2944 | 0.2087 | 1.3912 | 19.4714 | 14.0 | 0.63 |

The higher the ratio of acridinium to the antibody of the conjugate used in the immunoassay, the higher will be the chemiluminescent signal generated and, consequently, the higher will be the assay sensitivity achieved.

Substantially the same method as described above was used to prepare other conjugates that were used in the working examples. A succinimide ester derivatized from *N*¹⁰-(3-sulfopropyl)-*N*-(3-carboxypropyl)-acridinium-9-carboxamide can be used to prepare a CPSP-Acr-115B-151-423 conjugate used in some of the working examples (see Structure 2)

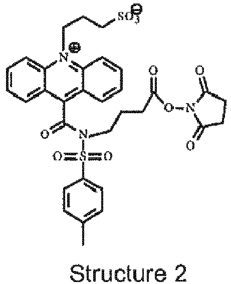

The following non-limiting working examples illustrate how the various embodiments of the method described herein can be carried out.

### EXAMPLE 1

This example illustrates how relatively large samples (1 mL) having a concentration of HIV-1 p24 antigen of lower than 1 pg/mL (0.2 pg/mL and 0.5 pg/mL) can produce a signal similar to that produced by using relatively small samples (0.1 mL) having concentrations of 2 pg/mL and 5 pg/mL when tested with the CPSP-Acr-115B-151-423 conjugate and magnetic particles coated with the anti-HIV-1 p24 monoclonal antibody 108-394-470 by means of magnetic particle processing.

The following materials were used in this example.
1. Magnetic microparticles coated with the anti-HIV-1 p24 monoclonal antibody 108-394-470. The magnetic microparticles were diluted to 0.2% solids in microparticle diluent.
2. CPSP-Acr-115B-151-423 conjugate
3. PRISM^{®} HIVAG transfer wash buffer
4. PRISM^{®} HIVAG conjugate wash buffer
5. Normal human plasma as negative control
6. HIV-1 p24 antigen stock (526 pg/mL) diluted with normal human plasma to make diluted samples of HIV-1 p24 antigen at the concentrations of 0.2, 0.5, 2, and 5 pg/mL
7. Nunc-Immuno™ 8-round bottom wells in the form of a strip
8. ARCHITECT^{®} pre-trigger solution
9. ARCHITECT^{®} trigger solution

The following equipment was used to perform the method of this example:
1. Luminometer, EG & G Berthold
2. Micro-well plate shaker, Lab-Line Instruments
3. Magnetic micro-well plate separator
4. Eight well micro-well plate suction head connected to vacuum
5. Multiple well pipette
6. Clay Adams Brand Nutator Rotator

The following method was employed to carry out the assay.
1. By means of a pipette, magnetic microparticles coated with the anti-HIV-1 p24 monoclonal antibody 108-394-470 (20 µL, 0.2% solids) were introduced into a micro-well of a micro-well plate for testing sample volumes of 0.1 mL. By means of a pipette, magnetic microparticles coated with the anti-HIV-1 p24 monoclonal antibody 108-394-470 (20 µL, 0.2% solids) were introduced into a test tube (12 x 75 mm, 5 mL polypropylene tube) for testing sample volumes of 1 mL.
2. Samples (0.1 mL) containing HIV-1 p24 antigen at concentrations of 2 pg/mL and 5 pg/mL were introduced into each micro-well of the micro-well plate by means of a pipette in two replicates. A new pipette tip was used each time. Samples (1 mL) containing HIV-1 p24 antigen at concentrations of 0.2 pg/mL and 0.5 pg/mL were introduced into each test tube in two replicates. The test tubes were placed in a test tube holder.
3. The contents of the micro-well plate and the test tubes in the test tube holder were incubated on a micro-well plate shaker for one hour at room temperature.
4. The micro-well plate was placed on a magnetic micro-well plate separator on a Nutator Rotator for two minutes until clear liquid was observed; the liquid was removed from the micro-wells by an 8-well suction head without disturbing the captured microparticles. The test tubes were placed on a magnetic separator for two minutes until clear liquid was observed; the liquid was removed manually from the test tubes by a suction head without disturbing the captured microparticles
5. Transfer wash buffer (100 µL) was introduced into each micro-well of the micro-well plate by a multiple-channel pipette. Transfer wash buffer (100 µL) was introduced into each test tube by means of a pipette.
6. The micro-well plate and the test tubes in the test tube holder were placed on a micro-well plate shaker for one minute to disperse magnetic microparticles and wash away non-specifically bound material.
7. Steps 4, 5, and 6 were repeated three times to wash away non-specifically bound material.
8. After the last washing step, the CPSP-Acr-115B-151-423 conjugate (50 µL, 60 ng/mL) was introduced into the micro-wells of the micro-well plate and the test tubes. The contents of the micro-well plate and the test tubes in the test tube holder were incubated on a plate shaker for one hour at room temperature.
9. Steps 4, 5, 6, and 7 were repeated three times to wash away unbound CPSP-Acr-115B-151-423 conjugate with conjugate wash buffer.
10. After the third washing, liquid containing magnetic microparticles from each micro-well of the micro-well plate was transferred into the micro-wells of a white flat bottom of Dynex microlite 2 plate, then the plate was placed on a magnetic micro-well plate separator on a Nutator rotator for two minutes until clear liquid was observed; the liquid was removed by an 8-well suction head without disturbing the captured microparticles. After the third washing, liquid containing magnetic microparticles from each test tube was transferred into the micro-wells of a white flat bottom of Dynex microlite 2 plate, then the plate was placed on a magnetic micro-well plate separator on a Nutator rotator for two minutes until clear liquid was observed; the liquid was removed by an 8-well suction head without disturbing the captured microparticles
11. ARCHITECT^{®} pre-trigger solution (40 µL) was introduced into each micro-well of the Dynex microlite 2 plates and the contents of the plates were incubated on a micro-well plate shaker for 10 minutes at room temperature to release the CPSP-Acr-115B-151-423 conjugate from the magnetic particles into the pre-trigger solution.
12. The chemiluminescence signal was read in a micro-well plate chemiluminescence reader (luminometer) by injecting of ARCHITECT^{®} trigger solution (120 µL) into each micro-well of the Dynex microlite 2 plates. The readings are given in Relative Light Units (RLU).

The results are shown in TABLE IV.

**TABLE IV**

| Concentration of HIV-1 p24 antigen (0.1 mL of sample) | Rep 1 (RLU) | Rep 2 (RLU) | Average RLU | S/N (Signal to Noise Ratio) |
|---|---|---|---|---|
| Negative control | 91 | 88 | 90 | |
| 2.0 pg/mL | 371 | 453 | 412 | 4.6 |
| 5.0 pg/mL | 933 | 901 | 917 | 10.2 |
| | | | | |

| Concentration of HIV-1 p24 antigen (1 mL of sample) | | | | |
|---|---|---|---|---|
| Negative control | 88 | 96 | 92 | |
| 0.2 pg/mL | 409 | 460 | 435 | 4.7 |
| 0.5 pg/mL | 805 | 1049 | 927 | 10.1 |

The data indicate that an increase in the volume of the sample by a factor of 10 can potentially increase the signal by a significant factor, thereby suggesting the use of a large volume of sample can improve the sensitivity of an immunoassay for HIV-1 p24 antigen.

### EXAMPLE 2

This example illustrates the determination of the concentration of HIV-1 p24 antigen at concentrations ranging from 0 to 1.6 pg/mL in samples by capturing HIV-1 p24 antigen from samples (2 mL) by means of a KingFisher™ mL magnetic particle processor using magnetic particles coated with the anti-HIV-1 p24 monoclonal antibody 120A-270-1068. CPSP-Acr-115B-151-423 conjugate was used to provide the label.

The following materials used in this example:
1. Magnetic microparticles coated with the anti-HIV-1 p24 monoclonal antibody 120A-270-1068. The magnetic microparticles were diluted to 0.25 % solids in microparticle diluent.
2. CPSP-Acr-115B-151-423 conjugate. The concentration of the conjugate was 50 ng/mL in conjugate diluent; the conjugate was filtered through filter disk (0.1 micrometer).
3. Microparticle diluent
4. HIV-1 p24 antigen stock (27.5 pg/mL) diluted with normal human plasma to make a series of diluted samples having concentrations of antigen of (a) 0.1 pg/mL (b) 0.2 pg/mL, (c) (d) 0.4 pg/mL, (d) 0.8 pg/mL, and (e) 1.6 pg/mL
5. Normal human plasma (as negative control)
6. ARCHITECT^{®} pre-trigger solution
7. ARCHITECT^{®} trigger solution
8. PRISM^{®} HIVAG transfer wash buffer
9. 10X specimen diluent buffer

The following pipettes were used in this example:
P5000, P1000, P200, P20, P2

The number following the letter "P" is the maximum volume, in microliters, that can be transferred by the subject pipette. The lower the value of maximum volume, the more accurate will be the volume delivered by the pipette.

The following equipment was used in this example:
1. KingFisher™ mL_magnetic particle processor
2. Micro-well plate luminometer; EG & G Berthold

The following sample setup was used in this example:
1. 10X specimen diluent buffer (0.11 mL) was placed in the tube A and in the tube B of the KingFisher™ mL magnetic particle processor.
2. Samples (1 mL) were added to the tubes A and B KingFisher™ mL magnetic particle processor. Samples were normal human plasma or HIV-1 p24 antigen diluted in normal human plasma.
3. Magnetic particles coated with the anti-HIV-1 p24 monoclonal antibody 120A-270-1068 (20 µL) were diluted to 0.25% solids and placed in the tube A of the KingFisher™ mL magnetic particle processor.
4. CPSP-Acr-115B-151-423 conjugate (0.1 mL, 50 ng/mL) was placed in the tube C of the KingFisher™ mL magnetic particle processor.
5. Wash buffer (1 mL) was placed in the tube D of the KingFisher™ mL magnetic particle processor.
7. ARCHITECT^{®} pre-trigger solution (60 µL) was placed in the tube E of the KingFisher™ mL magnetic particle processor.

The following table shows the distribution of materials among the five tubes of the KingFisher™ mL magnetic particle processor for performing a sandwich immunoassay for determining the concentration of HIV-1 p24 antigen.

**TABLE V**

| | Tube A (microliters) | Tube B (microliters) | Tube C (microliters) | Tube D (microliters) | Tube E (microliters) |
|---|---|---|---|---|---|
| 10X sample particle diluent | 110 | 110 | | | |
| Sample | 1000 | 1000 | | | |
| Magnetic microparticles coated with anti-HIV-1 monoclonal antibody 120A-270-1068 | 20 | | | | |
| CPSP-Acr-115B-151-423 conjugate | | | 100 | | |
| Wash buffer | | Wash* | | 1000 | |
| ARCHITECT^{®} pre-trigger solution | | | | | 60 |

| | | | | | |
|---|---|---|---|---|---|
| *The sample was also used as the wash buffer | | | | | |

The following assay protocol was used in this example:
1. Magnetic particles coated with the anti-HIV-1 p24 monoclonal antibody 120A-270-1068 were incubated at room temperature with the sample for 20 minutes with agitation in the tube A of the KingFisher™ mL magnetic particle processor.
2. Magnetic particles having captured HIV-1 p24 antigen were transferred from the tube A to the tube B of the KingFisher™ mL magnetic particle processor and incubated for 20 minutes at room temperature with agitation.
3. Magnetic particles were transferred from the tube B of the KingFisher™ mL magnetic particle processor to the tube C of the KingFisher™ mL magnetic particle processor and incubated for 20 minutes at room temperature with agitation.
4. Magnetic microparticles were transferred from the tube C of the KingFisher™ mL magnetic particle processor back to the tube B of the KingFisher™ mL magnetic particle processor to wash away free acridinium-labeled conjugate for two minutes.
5. Magnetic particles were transferred from the tube B of the KingFisher™ mL magnetic particle processor to the tube D of the KingFisher™ mL magnetic particle processor to further wash away free acridinium-labeled conjugate for two minutes.
6. CPSP-Acr-115B-151-423 conjugate specifically bound to HIV-1 p24 antigen that had been captured on the magnetic particles were eluted in ARCHITECT^{®} pre-trigger solution (60 µL, pH 2.6) in the tube E of the KingFisher™ mL magnetic particle processor. After elution, magnetic particles were removed and released into the wash tube D.
7. Pre-trigger solution (50 µL) was transferred from the tube E of the KingFisher™ mL magnetic particle processor to the micro-wells of an opaque micro-well plate.
8. The chemiluminescence signal was read at the appropriate wavelength in a luminometer by injecting ARCHITECT^{®} trigger solution (150 µL) into each micro-well of the opaque micro-well plate.

The following table shows the results of the foregoing assays.

**TABLE VI**

| Conc. of HIV-1 p24 antigen (pg/mL) | Dark count (RLU) | Total count (RLU) | Net count (RLU) | Dark count (RLU) | Total count (RLU) | Net count (RLU) | Dark count (RLU) | Total count (RLU) | Net count (RLU) | Mean count (RLU) | S/N |
|---|---|---|---|---|---|---|---|---|---|---|---|
| 0 | 24 | 45 | 21 | 27 | 43 | 16 | 25 | 41 | 16 | 18 | |
| 0.1 | 23 | 95 | 72 | 23 | 79 | 56 | 22 | 74 | 52 | 64 | 3.6 |
| 0.2 | 22 | 109 | 87 | 22 | 126 | 104 | 25 | 121 | 96 | 96 | 5.3 |
| 0.4 | 22 | 225 | 203 | | | | | | | 203 | 11.3 |
| 0.8 | 25 | 250 | 225 | 22 | 284 | 262 | | | | 244 | 13.5 |
| 1.6 | 24 | 839 | 815 | 22 | 930 | 908 | | | | 862 | 47.9 |

A signal to noise ratio in excess of 3, i.e., S/N greater than 3, enables a positive result to be distinguished form a negative result. According to TABLE VI, a concentration as low as 0.1 pg/mL will be deemed a positive result.

This example illustrates that an immunoassay performed by a KingFisher™ mL magnetic particle processor can provide (a) a high level of sensitivity, e.g., the ability to detect 0.1 pg/mL of HIV-1 p24 antigen, through the use of a large volume of sample, e.g., 2 mL; (b) an extremely low level of assay background because non-specifically bound conjugates remained in the tube and only specifically bound CPSP-Acr-115B-151-423 conjugate was eluted into the low pH (pH 2.6) pre-trigger solution for signal output; and (c) a high level of assay specificity because potential interfering components present in samples remained in the micro-wells and only HIV-1 p24 antigen bound to the magnetic microparticles were carried forward through the assay. In addition, before non-specifically bound conjugate is washed away from the magnetic microparticles with a final wash buffer, the sample in the sample tubes can be also used to wash away unbound conjugate from the magnetic microparticles after the incubation step involving the conjugate. This feature allows not only reserving more micro-wells for processing greater volumes of sample but also consuming lower quantities of wash buffer and generating lower quantities of liquid waste.

### EXAMPLE 3

This example illustrates the determination of the concentration of HIV-1 p24 antigen at concentrations ranging from 0 to 1.6 pg/mL in samples by capturing HIV-1 p24 antigen from samples (0.89 mL) through the use of magnetic particles coated with the anti-HIV-1 p24 monoclonal antibody 120A-270-1068 by means of a KingFisher™ magnetic particle processor. CPSP-Acr-115B-151-423 conjugate was used to provide the label.

The following materials used in this example:
1. Magnetic microparticles coated with the anti-HIV-1 p24 monoclonal antibody 120A-270-1068. The magnetic microparticles were diluted to 0.25 % solids in microparticle diluent.
2. CPSP-Acr-115B-151-423 conjugate. The concentration of the conjugate was 50 ng/mL in conjugate diluent; the conjugate was filtered through filter disk (0.1 micrometer).
3. ARCHITECT^{®} microparticle diluent
4. HIV-1 p24 antigen stock (77 pg/mL) diluted with in normal human plasma to make a series of diluted samples having concentrations of antigen of (a) 0.1 pg/mL (b) 0.2 pg/mL, (c) (d) 0.4 pg/mL, (d) 0.8 pg/mL, and (e) 1.6 pg/mL.
5. Normal human plasma as negative control
6. ARCHITECT^{®} pre-trigger solution
7. ARCHITECT^{®} trigger solution
8. PRISM^{®} HIVAG transfer wash buffer
9. 10X specimen diluent buffer

The following pipettes were used in this example:
P5000, P1000, P200, P20, P2

The number following the letter "P" is the maximum volume, in microliters, that can be transferred by the subject pipette. The lower the value of maximum volume, the more accurate will be the volume delivered by the pipette.

The following equipment was used in this example:
1. KingFisher™ magnetic particle processor, with eight (8) wells
2. Micro-well plate luminometer; EG & G Berthold

The following sample setup was used in this example:
1. 10X Specimen diluent buffer (20 µL) was placed in the wells A, B, C, D, E, and F of the KingFisher™ magnetic particle processor.
2. Sample (170 µL) was added to the well A and samples (180 µL) were added to the wells B, C, D, and E of the KingFisher™ magnetic particle processor. Samples were normal human plasma or HIV-1 p24 antigen diluted in normal human plasma and tested in two replicates.
3. Magnetic particles coated with the anti-HIV-1 p24 monoclonal antibody 120A-270-1068 (10 µL) were diluted to 0.25% solids and placed in the well A of the KingFisher™ magnetic particle processor.
4. CPSP-Acr-115B-151-423 conjugate (50 µL, 50 ng/mL) was placed in the well F of the KingFisher™ magnetic particle processor.
5. Wash buffer (200 µL) was placed in the well G of the KingFisher™ magnetic particle processor.
6. ARCHITECT^{®} pre-trigger solution (60 µL) was placed in the well H of the KingFisher™ magnetic particle processor.

TABLE VII illustrates the aforementioned set-up.

**TABLE VII**

| Ingredient | Well A (µL) | Well B (µL) | Well C (µL) | Well D (µL) | Well E (µL) | Well F (µL) | Well G (µL) | Well H (µL) |
|---|---|---|---|---|---|---|---|---|
| 10X specimen diluent buffer | 20 | 20 | 20 | 20 | 20 | | | |
| Sample | 170 | 180 | 180 | 180 | 180 | | | |
| Magnetic microparticles coated with monoclonal antibody 120A-270-1068 | 10 | | | | | | | |
| CPSP-Acr-115B-151-423 conjugate | | | | | | 50 | | |
| Wash buffer | | | Wash* | Wash* | Wash* | | 200 | |
| ARCHITECT^{®} pre-trigger solution | | | | | | | | 60 |

| | | | | | | | | |
|---|---|---|---|---|---|---|---|---|
| *The wash buffer consisted of the contents of the well | | | | | | | | |

The following assay protocol was used in this example:
1. Magnetic particles coated with the anti-HIV-1 p24 monoclonal antibody 120A-270-1068 were incubated at room temperature with the sample for 10 minutes with agitation in the wells A, B, C, D, and E of the KingFisher™ magnetic particle processor sequentially.
2. Magnetic particles having captured HIV-1 p24 antigen were transferred from the well E to the well F of the KingFisher™ magnetic particle processor and incubated for 10 minutes at room temperature, with agitation.
3. Magnetic particles were transferred from the well F of the KingFisher™ magnetic particle processor to the wells C, D, and E sequentially to wash away unbound acridinium-labeled conjugate for two minutes with agitation.
4. Magnetic microparticles were transferred from the well E of the KingFisher™ magnetic particle processor to the well G of the KingFisher™ magnetic particle processor to further wash away unbound acridinium-labeled conjugate for two minutes with agitation.
5. Magnetic particles were eluted in of ARCHITECT^{®} pre-trigger solution (60 µL, pH 2.6) in the well H of the KingFisher™ magnetic particle processor.
6. Magnetic particles in ARCHITECT^{®} pre-trigger solution (50 µL) from the first replicate of tested samples were transferred from the well H of the KingFisher™ magnetic particle processor to the micro-wells of an opaque micro-well plate. The chemiluminescence signal was read in a luminometer by injecting ARCHITECT^{®} trigger solution (150 µL) into each well of the opaque micro-well plate when the microparticles were dispersed.
7. Magnetic particles in ARCHITECT^{®} pre-trigger solution (50 µL) from the second replicate of tested samples were transferred from the well H of the KingFisher™ magnetic particle processor to the micro-wells of an opaque micro-well plate. The micro-well plate was placed on a micro-well plate holder with magnetic bars to trap magnetic particles to form pellets on the bottoms of the micro-wells of the opaque micro-well plate. The chemiluminescence signal was read in a luminometer by injecting ARCHITECT^{®} trigger solution (150 µL) into each micro-well of the opaque micro-well plate when the microparticles were pelleted.

The following table shows the results of the foregoing assays.

**TABLE VIII**

| | Magnetic Particles Dispersed | | | | Magnetic Particles Pelleted | | | |
|---|---|---|---|---|---|---|---|---|
| Conc. of HIV-1 p24 antigen (pg/mL) | Dark count (RLU) | Total count (RLU) | Net count (RLU) | S/N | Dark count (RLU) | Total count (RLU) | Net count (RLU) | S/N |
| 0 | 26 | 58 | 32 | | 26 | 68 | 42 | |
| 0.1 | 28 | 96 | 68 | 2.1 | 30 | 118 | 88 | 2.1 |
| 0.2 | 30 | 131 | 101 | 3.2 | 27 | 160 | 133 | 3.2 |
| 0.4 | 28 | 226 | 198 | 6.2 | 29 | 277 | 248 | 5.9 |
| 0.8 | 30 | 312 | 282 | 8.8 | 31 | 530 | 499 | 11.9 |
| 1.6 | 31 | 664 | 613 | 19.2 | 30 | 971 | 941 | 22.4 |

The data in TABLE VIII show that dispersed magnetic particles quench 20-43% of the chemiluminescence signal as compared with the wells containing pelleted magnetic particles. The percent quench of the luminescence signal was determined by the equation (Net RLU of pelleted particles - Net RLU of dispersed particles) / (Net RLU of pelleted particles) * 100%. The data also indicate that the assay performed by a KingFisher™ magnetic particle processor with 0.89 mL of sample volume can measure a concentration of 0.2 pg/mL of HIV-1 p24 antigen present in the sample with a signal-to-noise ratio of 3.2.

### EXAMPLE 4

This example illustrates the feasibility of determining the concentration of HIV-1 p24 antigen at concentrations below 1 pg/mL in samples by using a combination of a KingFisher™ mL_magnetic particle processor as a sample processor to capture HIV-1 p24 antigen from 1.6 mL of sample through the use of magnetic microparticles coated with the anti-HIV-1 p24 monoclonal antibody 120A-270-1068. CPSP-Acr-115B-151-423 conjugate was used to provide the label. The concentration was determined by means of an ARCHITECT^{®} automated immunoassay analyzer. Four KingFisher™ tubes were used for binding steps, and the particles were released by a wash step in a fifth KingFisher™ tube, which contained particle diluent (200 µL).

The following materials were used in this example.
1. HIV-1 p24 antigen stock (77 pg/ml) diluted in negative human plasma to make a series of diluted samples having concentrations of antigen of (a) 1.8 pg/mL (b) 0.6 pg/mL, (c) 0.2 pg/mL, and (d) 0.1 pg/mL
2. KingFisher™ diluent
3. PRISM^{®} transfer wash buffer
4. ARCHITECT^{®} combo microparticle diluent, containing 13.5% sucrose in 50 mM Tris-HCl buffer, pH 8.0, 100 mM NaCl, 0.1% Proclin^{®} antimicrobial agent, 0.1% Nipasept^{®} antimicrobial agent, and 0.0005% Quinolone^{®} antimicrobial agent.
5. Reagent packs for ARCHITECT^{®} assays consisting of conjugate containing anti-HIV-1 p24 monoclonal antibody 115B-151-423; magnetic microparticles coated with the anti-HIV-1 p24 monoclonal antibody 120A-270-1068; and PRISM^{®} specimen diluent buffer

The following KingFisher™ mL magnetic particle processor set-up was used to carry out this example. Fifteen strips of KingFisher™ tubes, each strip containing five tubes, were used. The first tube in each strip is designated A. The second tube in each strip is designated B. The third tube in each strip is designated C. The fourth tube in each strip is designated D. The fifth tube in each strip is designated E.

KingFisher™ diluent (0.1 mL), magnetic microparticles (0.1 mL), and sample (0.8 mL) were added into each tube A. KingFisher™ diluent (0.1 mL) and sample (0.8 mL) were added into each tube B. PRISM^{®} transfer wash buffer (0.5 mL) was added into each tube C. PRISM^{®} transfer wash buffer (0.5 mL) was added into each tube D. Particle diluent (0.16 mL) was added into each tube E.

The process protocol was carried out as follows:
1. During the binding step of the KingFisher™ process, HIV-1 p24 antigen in the samples in the tube A was captured by magnetic particles coated with the anti-HIV-1 p24 monoclonal antibody 120A-270-1068. The binding step had a duration of 20 minutes.
2. The magnetic particles in the tube A were collected and released to the tube B to repeat the same binding step as described in the step 1 to capture additional HIV-1 p24 antigen from samples.
3. During the washing step, the magnetic particles in the tube B were collected and released to the tube C, which contained PRISM^{®} transfer wash buffer (0.5 mL), to wash away non-specifically bound material from the particles. The wash step had a duration of 40 seconds.
4. The magnetic particles in the tube C were collected and released to the tube D to repeat the washing step for an additional 40 seconds.
5. The magnetic particles in the tube D were collected and released to the tube E, which contained ARCHITECT^{®} particle diluent (0.16 mL).

The released microparticles in the three tubes E were transferred into one ARCHITECT^{®} sample cup. This step was performed for each sample. The assays for HIV-1 p24 antigen were conducted with an ARCHITECT^{®} reagent pack, which included a conjugate, particle diluent, and specimen diluent buffer. For a side-by-side comparison, the same set of HIV-1 p24 antigen samples not processed in the KingFisher™ mL magnetic particle processor was also tested by a regular ARCHITECT HIV-1 p24 assay using an ARCHITECT^{®} reagent pack that contained conjugate, microparticles, and specimen diluent buffer. All of the samples were tested in triplicate.

The ARCHITECT^{®} HIV-1 p24 assay was a two-step sandwich immunoassay for the detection of HIV-1 p24 antigen in human serum and plasma. In the first step, sample (100 µL), specimen diluent buffer (50 µL), and paramagnetic microparticles coated with the anti-HIV-1 p24 monoclonal antibody 120A-270-1068 (50 µL) were combined and incubated for 18 minutes at a temperature of 37 °C. HIV-1 p24 antigen present in the sample bound to the microparticles coated with the anti-HIV-1 p24 monoclonal antibody 120A-270-1068. After washing, the CPSP-Acr-115B-151-423 conjugate (50 µL) was added and the mixture was incubated for four (4) minutes at a temperature of 37 °C. Following another wash cycle, the pre-trigger solution and the trigger solution were added to the reaction mixture. The resulting chemiluminescent reaction was measured in Relative Light Units (RLU). A direct relationship exists between the amount of HIV-1 p24 antigen in the sample and the quantity of RLU detected by the ARCHITECT^{®} optical system.

In the following table, "KFD" represents KingFisher™ diluent, "p24" represents HIV-1 p24 antigen, "NC" represents negative control (negative human plasma), and "µparticles" represents magnetic microparticles. The sample processing map described in TABLE IX represents five samples, the concentrations of the samples being 0 pg/mL, 0.1 pg/mL, 0.2 pg/mL, 0.6 pg/mL, and 1.8 pg/mL of HIV-1 p24 antigen, that are processed by a KingFisher™ mL magnetic particle processor in triplicate for each sample in a processing format of two binding tubes (A and B), two washing tubes (C and D), and one releasing tube (E).

**TABLE IX**

| Strip number | Tube A | Tube B | Tube C | Tube D | Tube E | Collected µparticles |
|---|---|---|---|---|---|---|
| 1 | KFD (0.1 mL); p24 (1.8 pg; 0.8 mL); µparticles (0.1 mL) | KFD (0.1 mL); p24 (1.8 pg; 0.8 mL) | PRISM^{®} transfer wash (0.5 mL) | PRISM^{®} transfer wash (0.5 mL) | ARCHITECT^{®} particle diluent (0.16 mL) | Combined µparticles in 1E, 6E, and 11E in sample tube, run 3X |
| 2 | KFD (0.1 mL); p24 (0.6 pg; 0.8 mL); µparticles (0.1 mL) | KFD (0.1 mL); p24 (0.6 pg; 0.8 mL) | PRISM^{®} transfer wash (0.5 mL) | PRISM^{®} transfer wash (0.5 mL) | ARCHITECT^{®} µparticle diluent (0.16 mL) | Combined µparticles in 2E, 7E, and 12E in sample tube, run 3X |
| 3 | KFD (0.1 mL); p24 (0.2 pg; 0.8 mL); µparticles (0.1 mL) | KFD (0.1 mL); p24 (0.2 pg; 0.8 mL) | PRISM^{®} transfer wash (0.5 mL) | PRISM^{®} transfer wash (0.5 mL) | ARCHITECT^{®} µparticle diluent (0.16 mL) | Combined particles in 3E, 8E, and 13E in sample tube, run 3X |
| 4 | KFD (0.1 mL); p24 (0.1 pg: 0.8 mL); µparticles (0.1 mL) | KFD (0.1 mL); p24 (0.1 pg; 0.8 mL) | PRISM^{®} transfer wash (0.5 mL) | PRISM^{®} transfer wash (0.5 mL) | ARCHITECT^{®} µparticle diluent (0.16 mL) | Combined particles in 4E, 9E, and 14E in sample tube, run 3X |
| 5 | KFD (0.1 mL); NC (0.8 mL); µparticles (0.1 mL) | KFD (0.1 mL); NC (0.8 mL) | PRISM^{®} transfer wash (0.5 mL) | PRISM^{®} transfer wash (0.5 mL) | ARCHITECT^{®} µparticle diluent (0.16 mL) | Combined µparticles in 5E, 10E, and 15E in sample tube, run 3X |

| Strip number | Tube A | Tube B | Tube C | Tube D | Tube E | |
|---|---|---|---|---|---|---|
| 6 | KFD (0.1 mL); p24 (1.8 pg; 0.8 mL); µparticles (0.1 mL) | KFD (0.1 mL); p24 (1.8 pg; 0.8 mL) | PRISM^{®} transfer wash (0.5 mL) | PRISM^{®} transfer wash (0.5 mL) | ARCHITECT^{®} particle diluent (0.16 mL) | |
| 7 | KFD (0.1 mL); p24 (0.6 pg; 0.8 mL); µparticles (0.1 mL) | KFD (0.1 mL); p24 (0.6 pg; 0.8 mL) | PRISM^{®} transfer wash (0.5 mL) | PRISM^{®} transfer wash (0.5 mL) | ARCHITECT^{®} particle diluent (0.16 mL) | |
| 8 | KFD (0.1 mL); p24 (0.2 pg; 0.8 mL); µparticles (0.1 mL) | KFD (0.1 mL); p24 (0.2 pg: 0.8 mL) | PRISM^{®} transfer wash (0.5 mL) | PRISM^{®} transfer wash (0.5 mL) | ARCHITECT^{®} particle diluent (0.16 mL) | |
| 9 | KFD (0.1 mL); p24 (0.1 pg; 0.8 mL); µparticles (0.1 mL) | KFD (0.1 mL); p24 (0.1 pg; 0.8 mL) | PRISM^{®} transfer wash (0.5 mL) | PRISM^{®} transfer wash (0.5 mL) | ARCHITECT^{®} µparticle diluent (0.16 mL) | |
| 10 | KFD (0.1 mL); NC (0.8 mL); particles (0.1 mL) | KFD (0.1 mL); NC (0.8 mL) | PRISM^{®} transfer wash (0.5 mL) | PRISM^{®} transfer wash (0.5 mL) | ARCHITECT^{®l} µparticle diluent (0.16 mL) | |
| 11 | KFD (0.1 mL); p24 (1.8 pg; 0.8 mL); µparticles (0.1 mL) | KFD (0.1 mL); p24 (1.8 pg; 0.8 mL) | PRISM^{®} transfer wash (0.5 mL) | PRISM^{®} transfer wash (0.5 mL) | ARCHITECT^{®} µparticle diluent (0.16 mL) | |
| 12 | KFD (0.1 mL); p24 (0.6 pg; 0.8 mL); µparticles (0.1 mL) | KFD (0.1 mL); p24 (0.6 pg; 0.8 mL) | PRISM^{®} transfer wash (0.5 mL) | PRISM^{®} transfer wash (0.5 mL) | ARCHITECT^{®} µparticle diluent (0.16 mL) | |
| 13 | KFD (0.1 mL); p24 (0.2 pg; 0.8 mL); particles (0.1 mL) | KFD (0.1 mL); p24 (0.2 pg; 0.8 mL) | PRISM^{®} transfer wash (0.5 mL) | PRISM^{®} transfer wash (0.5 mL) | ARCHITECT^{®} particle diluent (0.16 mL) | |
| 14 | KFD (0.1 mL); p24 (0.1 pg; 0.8 mL); particles (0.1 mL) | KFD (0.1 mL); p24 (0.1 pg; 0.8 mL) | PRISM^{®} transfer wash (0.5 mL) | PRISM^{®} transfer wash (0.5 mL) | ARCHITECT^{®} particle diluent (0.16 mL) | |
| 15 | KFD (0.1 mL); NC (0.8 mL); µparticles (0.1 mL) | KFD (0.1 mL); NC (0.8 mL) | PRISM^{®} transfer wash (0.5 mL) | PRISM^{®} transfer wash (0.5 mL) | ARCHITECT^{®} µparticle diluent (0.16 mL) | |

The results are shown in TABLES X and XI.

**TABLE X**

| Sample concentration | Mean RLU | Stan-dard deviation | %CV | S/N | Net RLU | | Rep 1 Raw RLU | Rep 2 Raw RLU | Rep 3 Raw RLU |
|---|---|---|---|---|---|---|---|---|---|
| Normal human plasma 11025 | 143.7 | 21.1 | 14.7 | 1.0 | 0 | | 133 | 130 | 168 |
| 0.1 pg/mL | 249.3 | 16.7 | 6.7 | 1.7 | 106 | | 268 | 236 | 244 |
| 0.2 pg/mL | 449.7 | 70.7 | 15.7 | 3.1 | 306 | | 403 | 415 | 531 |
| 0.6 pg/mL | 1317.0 | 573.3 | 43.5 | 9.2 | 1173 | | 956 | 1017 | 1978 |
| 1.8 pg/mL | 3366.3 | 587.7 | 17.5 | 23.4 | 3223 | | 2983 | 3073 | 4043 |

**TABLE XI**

| Sample concentration | Mean RLU | Standard deviation | %CV | S/N | Net RLU | | Rep 1 Raw RLU | Rep 2 Raw RLU | Rep 3 Raw RLU |
|---|---|---|---|---|---|---|---|---|---|
| Normal human plasma 11025 | 313.0 | 7.0 | 2.2 | 1.0 | 0 | | 308 | 310 | 321 |
| 0.1 pg/mL | 331.3 | 12.1 | 3.6 | 1.1 | 18 | | 330 | 320 | 344 |
| 0.2 pg/mL | 395.3 | 14.2 | 3.6 | 1.3 | 82 | | 403 | 404 | 379 |
| 0.6 pg/mL | 570.0 | 6.2 | 1.1 | 1.8 | 257 | | 568 | 577 | 565 |
| 1.8 pg/mL | 1115.0 | 60.0 | 5.4 | 3.6 | 802 | | 1075 | 1086 | 1184 |

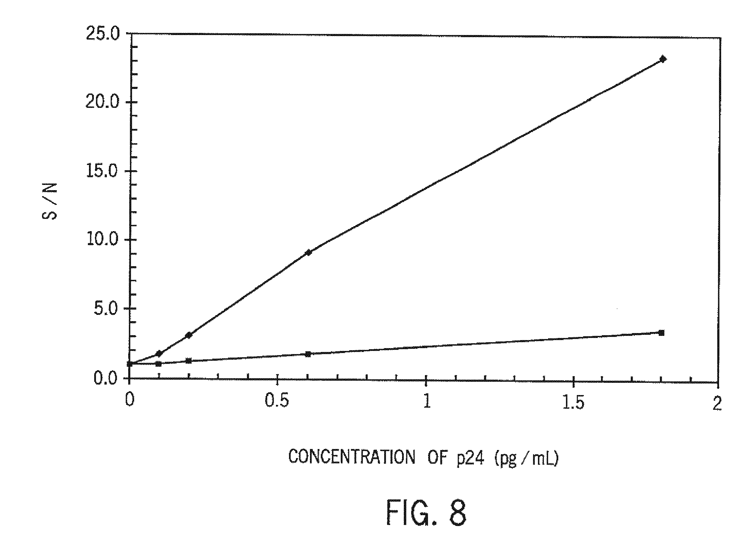

The data in TABLE X illustrate the use of KingFisher™ mL magnetic particle processor to prepare samples (a total of 1.6 mL) for an immunoassay performed on an ARCHITECT^{®} immunoassay analyzer. The data in TABLE XI illustrate samples (100 µL) run in the normal manner on an ARCHITECT^{®} immunoassay instrument (i.e., untreated by the KingFisher™ mL magnetic particle processor). If the signal to noise ratio exceeds 3, a positive sample can be distinguished from a negative sample. The data in both TABLES X and XI show that as the concentration of antigen increased, the signal, as expressed in RLU, also increased. However, the signal-to-noise ratio achieved through the use of the KingFisher™ mL magnetic particle processor far exceeded the signal-to-noise ratio achieved without the use of the KingFisher™ mL magnetic particle processor. The data are shown graphically in FIG. 8. FIG. 8 shows a plot of signal-to-noise ratio for samples analyzed in an ARCHITECT^{®} automated immunoassay analyzer both without previous treatment by inverse magnetic particle processing (indicated by squares) and with previous treatment by inverse magnetic particle processing (indicated by diamonds). The RLU readings can be calibrated to indicate the concentration of antigen in a sample. Standard deviation, %CV, and signal-to-noise ratio were within acceptable ranges.

### EXAMPLE 5

This example illustrates how an ultrasensitive immunoassay can be run on a PRISM^{®} automated immunoassay analyzer after the KingFisher™ mL magnetic microparticle processor is used to process a sample having a large volume. The PRISM^{®} automated immunoassay analyzer is commercially available from Abbott Laboratories for blood screening using latex microparticles and chemiluminescence detection technology. The following materials were used in this example.
1. Magnetic microparticles coated with the anti-HIV-1 p24 monoclonal antibody 120A-270-1068. The magnetic microparticles were diluted to 0.5 % solids in microparticle diluent.
2. SPSP-Acr-115B-151-423 conjugate
3. ARCHITECT® magnetic diluent
4. HIV-1 p24 antigen stock (77 pg/ml) diluted to the concentration of 0.2 pg/mL with normal human plasma for the KingFisher™ mL magnetic microparticle process; HIV-1 p24 antigen stock (77 pg/ml) diluted to the concentration of 4.2 pg/mL for regular PRISM^{®} control in the assay
5. Normal human plasma as negative control
6. PRISM^{®} activator diluent
7. PRISM^{®} activator concentrate
8. PRISM^{®} HIVAG transfer wash
9. Final wash buffer 7G56D
10. 10X specimen diluent buffer
11. CML latex microparticles coated with anti-HIV-1 monoclonal antibody 120A-270-1068 (available from Abbott Laboratories, Abbott Park, IL)

The following pipettes were used in this example:
P2, P20, P200, P1000, P5000

The number following the letter "P" is the maximum volume, in microliters, that can be transferred by the subject pipette. The lower the value of maximum volume, the more accurate will be the volume delivered by the pipette.

The KingFisher™ mL magnetic microparticle processor and the PRISM^{®} automated analyzer were used to carry out this example.

Sample preparation for the KingFisher™ mL magnetic microparticle processor was carried out in a five tube apparatus as shown in TABLE XII. Magnetic microparticles coated with anti-HIV-1 monoclonal antibody 120A-270-1068 were pre-washed in elution buffer (25 mM glycine-HCl buffer containing 0.15% bovine serum albumin, pH 2.1) in the tube A for three minutes and pre-captured by the KingFisher™ magnetic rod to select highly magnetic particles. The captured particles were then transferred to sample tubes B, C, and D (each tube B, C, and D containing specimen diluent buffer (0.3 mL) and sample (0.7 mL) to capture HIV-1 p24 antigen for 15 minutes in each sample tube. Magnetic particles having HIV-1 p24 antigen bound thereto were transferred to the elution tube E, which contained elution buffer (175 µL). After HIV-1 p24 antigen was eluted from the magnetic particles for two minutes, magnetic particles coated with anti-HIV-1 p24 monoclonal antibody 120A-270-1068 were transferred to the tube D. The total time of preparation was approximately 60 minutes, including two minutes for each particle collection and transfer in each step.

**TABLE XII**

| Tube | Ingredient (amount) | Time (minutes) |
|---|---|---|
| A | Elution buffer (1 mL), bovine serum albumin (0.15%, pH 2.1), magnetic microparticles coated with anti-HIV-1 p24 monoclonal antibody 120A-270-1068 diluted in ARCHITECT^{®} particle diluent (50 µL) | 3 |
| B | Specimen diluent buffer (0.3 mL), sample (0.7 mL) | 15 |
| C | Specimen diluent buffer (0.3 mL), sample (0.7 mL) | 15 |
| D | Specimen diluent buffer (0.3 mL), sample (0.7 mL) | 15 |
| E | Elution buffer (175 µL) | 2 |

The following method was employed to carry out an assay for HIV-1 in a PRISM^{®} automated analyzer.
1. Glycine-HCl eluted HIV-1 p24 antigen (150 µL) was transferred in a sample well of the PRISM^{®} automated analyzer.
2. Latex microparticle coated with anti-HIV-1 p24 monoclonal antibody 120A-270-1068 (50 µL, 0.033% diluted in 0.2 M Tris buffer (13.6% sucrose)) was added to recapture HIV-1 p24 antigen for 18 minutes at a temperature of 37 °C.
3. HIVAG transfer wash buffer was dispensed to flush the reaction mixture into a fiberglass tab well on a PRISM^{®} sample tray to wash away unbound material from the latex microparticles
4. SPSP-Acr-115B-151-423 conjugate (50 µL, 25 ng/mL diluted in 7G46J diluent) was added at the conjugate station manually to react for 22 minutes at a temperature of 37 °C with HIV-1 p24 antigen that had been captured on the latex microparticles,
5. Final wash buffer (7G56D) was dispensed into the fiberglass tab well on the PRISM^{®} sample tray to wash away free conjugate from the latex microparticles
6. PRISM^{®} trigger solution was added to the into the fiberglass tab well to generate a chemiluminescent signal.

The current PRISM^{®} HIVAG assay protocol was satisfactory to obtain over 70% recovery rate. The results are shown in TABLE XV.

**TABLE XV**

| Assay | Rep 1 (RLU) | Rep 2 (RLU) | Rep 3 (RLU) | Mean (RLU) | S/N | Mean RLU of run |
|---|---|---|---|---|---|---|
| PRISM^{®} assay with KingFisher^{®} preparation | | | | | | |
| Negative control | 30 | 28 | | 29 | | 37.0 |
| 0.2 pg/mL, 2.1 mL, 3 wells | 114 | 95 | 113 | 107.3 | 3.7 | 114.0 |
| | | | | | | |
| Negative control | 37 | 84 | | 60.5 | | % recovery |
| 0.2 pg/mL, 2.1 mL, 3 wells | 126 | 125 | 115 | 122.0 | 2.0 | 72.5 |
| | | | | | | |
| Negative control | 18 | 25 | | 21.5 | | |
| 0.2 pg/mL, 2.1 mL, 3 wells | 112 | 105 | 121 | 112.7 | 5.2 | |
| | | | | | | |
| Regular PRISM^{®} assay (no KingFisher^{®} preparation) | Rep 1 (RLU) | Rep 2 (RLU) | Rep 3 (RLU) | Rep 4 (RLU) | Mean (RLU) | S/N |
| 0 pg/mL | 28 | 17 | 65 | 24 | 33.5 | |
| 4.2 pg/mL | 159 | 143 | 162 | 165 | 157.3 | 4.7 |

The data in TABLE XV show the regular PRISM^{®} assay had a signal-to-noise ratio of 4.7 while the signal-to-noise ratios achieved by means of a combination of inverse magnetic particle processing and the PRISM^{®} assay were 3.7, 2.0, and 5.2. However, it should be noted that the concentration of the antigen in the regular PRISM^{®} assay was 21 times greater than the concentration of the antigen in the assay combining inverse magnetic particle processing and the PRISM^{®} assay. In effect, the assay combining inverse magnetic particle processing and the PRISM^{®} assay provided a signal-to-noise ratio that was approximately nine (9) to twenty-three (23) times greater than that that provided by the regular PRISM^{®} assay.
Various modifications and alterations of this invention will become apparent to those skilled in the art without departing from the scope and spirit of this invention, and it should be understood that this invention is not to be unduly limited to the illustrative embodiments set forth herein.

The present invention further encompasses the following embodiments:
1. A method for determining the concentration of an analyte in a biological sample, the biological sample suspected of containing the analyte, the analyte comprising a first member of a specific binding pair, the method comprising the steps of:
   (a) providing a plurality of containers;
   (b) introducing a biological sample suspected of containing an analyte comprising a first specific binding member and magnetic microparticles coated with a second specific binding member into at least one container of the plurality of containers;
   (c) allowing the first specific binding member to react with the second specific binding member to form a complex comprising the magnetic microparticles;
   (d) collecting the complex formed in step (c) by means of inverse magnetic particle processing;
   (e) releasing the complex collected in step (d) into a container of the plurality of containers containing wash buffer by means of inverse magnetic particle processing;
   (f) washing the complex released in step (e) to wash away unbound first binding member and non-specifically bound components by means of inverse magnetic particle processing;
   (g) collecting the complex washed in step (f) by means of inverse magnetic particle processing;
   (h) introducing the complex into an automated immunoassay analyzer; and
   (i) determining the concentration of the analyte in the sample by means of the automated immunoassay analyzer.
2. The method of item 1, wherein the first specific binding member is an antigen.
3. The method of item 2, wherein the antigen is an HIV antigen.
4. The method of item 2, wherein the second specific binding member is an antibody specific for the antigen.
5. The method of item 4, wherein the antibody is specific for an HIV antigen.
6. The method of item 1, wherein a luminescent label is used to determine the concentration of the analyte.
7. The method of item 6, wherein the luminescent label is a moiety of a conjugate.
8. The method of item 7, wherein the conjugate comprises a moiety that specifically binds to one of the first specific binding member or the second specific binding member.
9. The method of item 6, wherein the conjugate is introduced into the automated immunoassay analyzer.
10. The method of item 6, wherein the luminescent label is a chemiluminescent label.
11. The method of item 1, wherein the plurality of containers comprises a plurality of tubes.
12. The method of item 1, wherein the plurality of containers comprises a plurality of micro-wells.

## Claims

1. A method for determining the concentration of an analyte in a biological sample, the biological sample suspected of containing the analyte, the analyte comprising a first member of a specific binding pair, the method comprising the steps of:
(a) providing a plurality of containers;
(b) introducing a biological sample suspected of containing an analyte comprising a first specific binding member and magnetic microparticles coated with a second specific binding member into at least one container of the plurality of containers;
(c) allowing the first specific binding member to react with the second specific binding member to form a complex comprising the magnetic microparticles;
(d) collecting the complex formed in step (c) by means of inverse magnetic particle processing;
(e) releasing the complex collected in step (d) into a container of the plurality of containers, said container containing either (i) wash buffer but no third specific binding member or (ii) a third specific binding member, said releasing carried out by means of inverse magnetic particle processing;
(f) if the container in step (e) contains the wash buffer but does not contain the third specific binding member, adding the third specific binding member to the container of step (e);
(g) allowing the third specific binding member to react with the complex released in step (e) to form a sandwich complex;
(h) collecting the sandwich complex formed in step (g) by means of inverse magnetic particle processing,
(i) releasing the sandwich complex collected in step (h) into a container of the plurality of containers, which container contains either (i) wash buffer, to wash away unbound third specific binding member, or (ii) sample, to wash away unbound third specific binding member, said releasing carried out by means of inverse magnetic particle processing;
(j) collecting the sandwich complex released in step (i) by means of inverse magnetic particle processing;
(k) releasing the sandwich complex collected in step (j) into a container of the plurality of containers by means of inverse magnetic particle processing, which container contains pre-trigger solution, thereby releasing the specifically bound third binding member;
(l) collecting the magnetic particles from the pre-trigger solution by means of inverse magnetic particle processing and releasing the magnetic microparticles into a container of the plurality of containers, which container contains wash buffer; and
(m)determining the concentration of the analyte in the sample by injecting trigger solution into the pre-trigger solution and reading the signal generated.

2. The method of claim 1, wherein the first specific binding member is an antigen.

3. The method of claim 2, wherein the antigen is an HIV antigen.

4. The method of claim 2, wherein the second specific binding member is an antibody specific for the antigen.

5. The method of claim 4, wherein the antibody is specific for an HIV antigen.

6. The method of claim 1, wherein a luminescent label is used to determine the concentration of the analyte.

7. The method of claim 6, wherein the luminescent label is a moiety of a conjugate.

8. The method of claim 7, wherein the conjugate comprises a moiety that specifically binds to one of the first specific binding member or the second specific binding member.

9. The method of claim 7, wherein the luminescent label is a chemiluminescent label.
